# EUROPÄISCHE PATENTANMELDUNG

(11) **EP 1 314 420 A1**
(43) Veröffentlichungstag der Anmeldung: **28.05.2003**
(21) Anmeldenummer: 01127915.5
(22) Anmeldetag: 23.11.2001
(51) Int. Cl.: A61K 7/48, A61K 35/78

(54) **Anti-Ageing Mittel**

(71) Anmelder: Cognis Iberia, S.L., 08755 Castellbisbal, (Barcelona) (ES)
(72) Erfinder: Arias, Carmen, 08190 Sant Cugat del Vallés, Barcelona (ES); Rull Prous, Santiago, 08034 Barcelona (ES); Fabry, Dr. Bernd,, 41352 Korschenbroich (DE)
(74) Vertreter: Fabry, Bernd, Dr.

(57) **Zusammenfassung**

Vorgeschlagen werden neue Anti-Ageing-Mittel, welche sich dadurch auszeichnen, dass sie einen wirksamen Gehalt an Pflanzenextrakten der Gattung Vaccinium enthalten.

## Beschreibung

### Gebiet der Erfindung

Die Erefindung befindet sich auf dem Gebiet der Kosmetik und betrifft neue Zubereitungen gegen den Einfluss von Alterung und Umweltgiften auf Haut und Haare, die sich durch einen wirksamen Gehalt an Vaccinium-Extrakten auszeichnen sowie die Verwendung der Extrakte gegen eine Vielzahl von Effekten, die Haut und Haare schädigen.

### Stand der Technik

Schon in den Zeiten der Antike hat der Wunsch nach ewiger Schönheit und Jugend bestanden. Während von Kleopatra überliefert ist, dass sie regelmäßig ein Bad in Eselsmilch zu nehmen pflegte - heute wissen wir um die Wirkung der darin enthaltenen Milchproteine - mussten weniger begüterte Frauen, darauf hoffen, dass ihr Wunsch von den Göttern erhört würde. Es steht zu vermuten, dass dies nur selten von Erfolg gekrönt worden ist. Heutzutage ist jugendliches Aussehen und eine faltenarme Haut kein Privileg einiger weniger, sondern steht trotz teilweise erheblicher Preisunterschiede bei den Präparaten grundsätzlich allen Frauen zur Verfügung. Auch wenn die kosmetische Chemie keine Wunder vollbringen kann, hat aber gerade in den letzten Jahren das Wissen um die biochemischen Abläufe in den Zellen von Haut und Haaren sprunghaft zugenommen. Konsequenterweise haben sich dadurch natürlich Ansätze ergeben, auf welche Weise man Schädigungen, die durch natürliche Alterung oder Umwelteinflüsse eintreten, verhindern oder beheben kann. In gleicher Weise sind jedoch auch die Anforderungen gestiegen, die die Verbraucherinnen (und zunehmend auch Verbraucher) an solche sogenannten "Anti-Ageing Mitteln" stellen. Ganz abgesehen davon, dass die Zubereitungen grundsätzlich einen pflegenden Charakter aufweisen sollen und optimal haut- und gegebenenfalls schleimhautverträglich sein müssen, sollen sie vor UV-Strahlung und Umweltgiften schützen, das Immunsystem stimulieren und antiinflammatorisch wirksam sein.

In diesem Zusammenhang sei beispielsweise auf die Deutsche Patentanmeldung **DE 3027933 A1** (Inverni Della Beffa) verwiesen, aus der pharmazeutische Zubereitungen für die topische Anwendung auf der Haut bekannt sind, die als Wirkstoff Vaccinium-Extrakte mit hohem Gehalt an Anthocyanosiden enthalten. Diese Mittel beleben den Blutkreislauf und werden zur Wundbehandlung eingesetzt. Aus der Europäischen Patentanmeldung **EP 0953353 A1** (Laboratoires Pharmascience) ist die Verwendung von Vaccinium-Extrakten als Anti-Urease-Agentien bekannt. Gegenstand der Internationalen Patentanmeldung **WO/01/45648** (L'Oréal) ist die Verwendung von Extrakten von Pflanzen der Gattung Vaccinium zur Verminderung oder Inhibierung der Glykation von Proteinen in der Haut oder verwandten Strukturen. In der Internationalen Patentanmeldung **WO99/55352** (Avon) werden Zubereitung zur Hautweißung vorgeschlagen, die eine synergistische Kombination vom Extrakt der Lakritzwurzel und einem Antioxidants, beispielsweise Vaccinium-Extrakt. Dekorative Kosmetika mit einem Gehalt an Vaccinium-Extrakten und Ölkörpern werden in der internationalen Patentanmeldung WO01/45662 (Laboratoires Sérobiologiques) vorgeschlagen. Gegenstand der Französischen Patentschrift **FR 2659556 B1** (Mu Lab Eurl) sind Zubereitungen mit Extrakten von Blaubeeren und Arnika. In der Französischen Patentschrift **FR 2612775 B1** (Thorel) wird über Hautbehandlungsmittel berichtet, die neben Vitamin A noch Quellen für Schwefel, Mangan und Magnesium enthalten, Vorzugsweise enthalten diese Zubereitungen Blaubeerextrakte und Ascorbinsäure. Schließlich werden in der Internationalen Patentanmeldung **WO 96/30033** Vaccinium-Extrakte in geeigneten Darreichungsformen, beispielsweise als Tabletten oder Kapseln, vorgeschlagen, die zur Reinigung beispielsweise von medizinischen Bestecken eingesetzt werden.

Die Aufgabe der vorliegenden Erfindung hat folglich darin bestanden, neue Anti-Ageing-Mittel mit einem Wirkstoff zur Verfügung zu stellen, der das oben beschriebene komplexe Anforderungsprofil erfüllt. Im Hinblick auf die BSE-Diskussion besteht ferner der Wunsch, dass es sich bei diesem "Multifunktionswirkstoffen" um ein pflanzliches Produkt handelt.

### Beschreibung der Erfindung

Gegenstand der Erfindung sind Anti-Ageing-Mittel, welche sich dadurch auszeichnen, dass sie einen wirksamen Gehalt an Pflanzenextrakten der Gattung Vaccinium enthalten.

Überraschenderweise wurde gefunden, dass Pflanzenextrakte der Gattung Vaccinium Haut und Haare vor dem Angriff freier Radikale schützt. Die antioxidative Wirksamkeit liegt dabei deutlich über der von Tocopherol und Butylhydroxytoluol und in der gleichen Größenordnung wie Vitamin C. Die Extrakte sind auch gegen UV-A und UV-B-Strahlung wirksam und vermindern insbesondere die Freisetzung von Matrix-Metalloproteinasen (MMP), Lactat Dehydrogenase (LDH) und Prostaglandinen. Sie besitzen ferner anti-inflammatorische Wirkung, da sie die Ausschüttung von Proteasen, speziell Collagenasen, reduzieren und den Atmungsausbruch menschlicher Granulocyten vermindern, ohne diese zu schädigen. Die Extrakte stimulieren das Immunsystem der Haut, das Wachstum und die Regeneration der Fibroblasten sowie die Synthese von Glucose-6-phosphat Dehydrogenase (G6PD). Schließlich fördern sie zusätzlich auch noch die Lipolyse von Fetten in der Haut

Ein weiterer Gegenstand der Erfindung betrifft daher die Verwendung von Pflanzenextrakten der Gattung Vaccinium als Mittel zum Schutz von Haut und Haaren gegen Alterung und Umwelteinflüsse, in denen sie in Mengen von 0,001 bis 1, vorzugsweise 0,005 bis 0,01 Gew.-% - bezogen auf den Gehalt an aktiven Wirkstoffen - enthalten sein können.

### Vaccinium-Extrakte

Unter der Gattung Vaccinium versteht der Botaniker eine Gruppe von rund 450 Spezies, von denen *Vaccinium myrtyllus*, die gemeine Heidel- oder Blaubeere, die wichtigste ist. Weitere bekannte Spezies sind *Vaccinium angustifollium*, *Vaccinium arboreum*, *Vaccinium arctostaphylos, Vaccinium caespitosum, Vaccinium corymbosum, Vaccinium hirsutum, Vaccinium macrocarpum, Vaccinium ovatum, Vaccinium oxycoccus, Vaccinium stamineum, Vaccinium uliginosum* sowie *Vaccinium vitis idaea*. Als aktive Wirkstoffe enthalten die Vaccinium-Extrakte eine Mischung aus mindestens 15 verschiedenen Anthocyanosiden , wie beispielsweise dem folgenden:

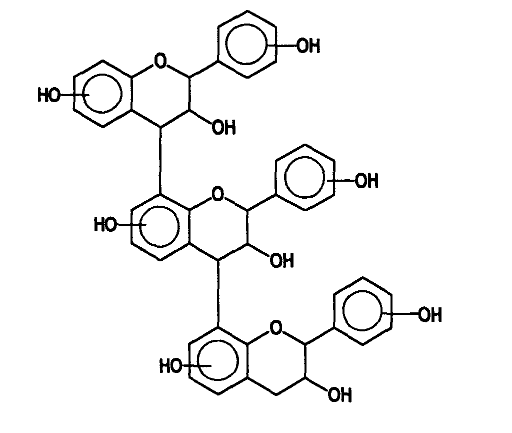

In der Regel enthalten die Vaccinium-Extrakte 20 bis 25 Gew.-% Anthocyanoside, 5 bis 10 Gew.-% Tannine sowie in geringen Mengen verschiedene Alkaloide (z.B. Myrtin und Epimyrtin), Phenolsäuren sowie Glycoside mit Quercitrin, Isoquercitrin und Hyperosid.

### Extraktion

Die Herstellung der Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluss die in einem Soxhlet-Extraktor durchgeführt wird, die dem Fachmann geläufig und im Prinzip alle anwendbar sind, sei der Einfachheit halber beispielsweise auf **Hagers Handbuch der Pharmazeutischen Praxis,** (5. Auflage, Bd. 2, S. 1026-1030, Springer Verlag, Berlin-Heidelberg-New-York 1991) verwiesen. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprühoder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### Anti-Ageing-Mittel

Die erfindungsgemäßen Mittel liegen üblicherweise als Cremes, Gele, Lotionen, alkoholische und wäßrig/alkoholische Lösungen, Emulsionen, Wachs/Fett-Massen, Stiftpräparate, Puder oder Salben vor. Sie können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, a-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, A-midseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, A-cylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, a-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1),** insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
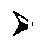 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
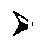 Alkyl- und/oder Alkenyloligoglykoside mit 8 bis 22 Kohlenstoffatomen im Alk(en)ylrest und deren ethoxylierte Analoga;
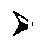 Anlagerungsprodukte von 1 bis 15 Mol Ethylenoxid an Ricinusöl und/oder gehärtetes Ricinusöl;
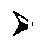 Anlagerungsprodukte von 15 bis 60 Mol Ethylenoxid an Ricinusöl und/oder gehärtetes Ricinusöl;
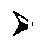 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
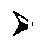 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
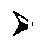 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
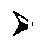 Mono-, Di- und Trialkylphosphate sowie Mono-, Di- und/oder Tri-PEG-alkylphosphate und deren Salze;
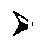 Wollwachsalkohole;
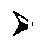 Polysiloxan-Polyalkyl-Polyether-Copolymere bzw. entsprechende Derivate;
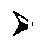 Block-Copolymere z.B. Polyethylenglycol-30 Dipolyhydroxystearate;
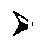 Polymeremulgatoren, z.B. Pemulen-Typen (TR-1,TR-2) von Goodrich;
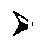 Polyalkylenglycole sowie
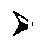 Glycerincarbonat.

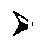 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

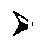 Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer-Zuckerrest-glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

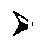 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

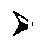 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

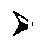 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

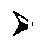 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

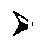 Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vlnylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolldon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie-amino, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
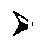 3-Benzylidencampher bzw. 3-Benzylidennorcampher und dessen Derivate, z.B. 3-(4-Methylbenzyliden)campher wie in der **EP 0693471 B1** beschrieben;
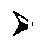 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
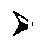 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
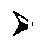 Ester der Salicylsäure, vorzugsweise Salicylsäure-2-ethylhexylester, Salicylsäure-4-isopropylbenzylester, Salicylsäurehomomenthylester;
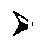 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
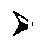 Ester der Benzalmalonsäure, vorzugsweise 4-Methoxybenzmalonsäuredi-2-ethylhexylester;
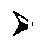 Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
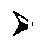 Propan-1,3-dione, wie z.B. 1-(4-tert.Butylphenyl)-3-(4'methoxyphenyl)propan-1,3-dion;
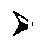 Ketotricyclo(5.2.1.0)decan-Derivate, wie in der **EP 0694521 B1** beschrieben.

Als wasserlösliche Substanzen kommen in Frage:
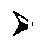 2-Phenylbenzimidazol-5-sulfonsäure und deren Alkali-, Erdalkali-, Ammonium-, Alkylammonium-, Alkanolammonium- und Glucammoniumsalze;
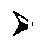 Sulfonsäurederivate von Benzophenonen, vorzugsweise 2-Hydroxy-4-methoxybenzophenon-5-sulfonsäure und ihre Salze;
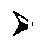 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. a-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. a-Hydroxyfettsäuren, Palmitinsäure, Phytinsäure, Lactoferrin), a-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, a-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

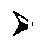 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

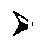 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

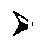 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

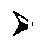 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
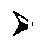 adstringierende Wirkstoffe,
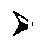 Ölkomponenten,
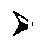 nichtionische Emulgatoren,
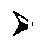 Coemulgatoren,
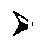 Konsistenzgeber,
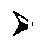 Hilfsstoffe wie z. B. Verdicker oder Komplexierungsmittel und/oder
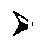 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
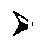 entzündungshemmende, hautschützende oder wohlriechende ätherische Öle,
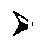 synthetische hautschützende Wirkstoffe und/oder
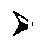 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische-Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw.

Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
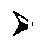 Glycerin;
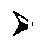 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
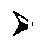 technische Oligoglyceringemische mit einem Eigenkondensationsgrad von 1,5 bis 10 wie etwa technische Diglyceringemische mit einem Diglyceringehalt von 40 bis 50 Gew.-%;
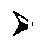 Methyolverbindungen, wie insbesondere Trimethylolethan, Trimethylolpropan, Trimethylolbutan, Pentaerythrit und Dipentaerythrit;
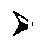 Niedrigalkylglucoside, insbesondere solche mit 1 bis 8 Kohlenstofen im Alkylrest, wie beispielsweise Methyl- und Butylglucosid;
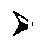 Zuckeralkohole mit 5 bis 12 Kohlenstoffatomen, wie beispielsweise Sorbit oder Mannit,
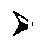 Zucker mit 5 bis 12 Kohlenstoffatomen, wie beispielsweise Glucose oder Saccharose;
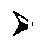 Aminozucker, wie beispielsweise Glucamin;
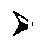 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Y-lang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

In den nachfolgenden Versuchen wurde als Testsubstanz ein Extrakt von *Vaccinium myrtyllus* mit einem Aktivsubstanzgehalt von 25 Gew.-% der Firma Cognis eingesetzt

### A. Wirksamkeit gegenüber freien Radikalen

Die Wirksamkeit der Testsubstanzen gegen freie Radikale wurde nach verschiedenen Methoden sowohl chemisch als auch biochemisch untersucht:
- Methode A: In einem ersten Verfahren wurde Diphenylpicrylhydrazyl (DPPH°) eingesetzt, ein verhältnismäßig stabiles Radikal, welches eine purpurfarbene Lösung ergibt. Bestimmt wurde die optische Dichte (DO) bei 513 nm.
- Methode B: In Gegenwart von Eisen(II)-ionen und EDTA wurden aus Wasserstoffperoxid Hydroxylradikale freigesetzt und zur Oxidation von Desoxyribose verwendet. Das Oxidationsprodukt bildet mit Thiobarbitursäure eine pinkfarbene Verbindung. Dessen Konzentration korrespondiert mit der optischen Dichte bei 532 nm. Untersucht wurde, ob in Gegenwart der Testprodukte weniger Desoxyribose oxidiert, d.h. weniger freie Radikale freigesetzt werden.
- Methode C: Der zuvor geschilderte Versuch wurde in Abwesenheit von EDTA untersucht um die Eignung der Testsubstanzen, inaktive Eisenkomplexe zu bilden, zu überprüfen.
- Methode D: Xanthin Oxidase ist ein Enzym welches infolge oxidativen Stresses ausgeschüttet wird und den Zerfall der Purinbasen Adenin und Guanin in Uronsäure und Superoxidanionen katabolisiert. Letztere dismutieren spontan oder in Gegenwart von Superoxid Dismutase in Wasserstoffperoxid und Sauerstoff. Die Menge an Superoxidanion kann durch NBT Reduktion über die optische Dichte bei 490 nm bestimmt werden. Untersucht wurde, ob in Gegenwart der Testsubstanzen weniger Superoxidanionen generiert bzw. mehr Anionen zerstört werden.

Die Ergebnisse sind in Tabelle 1 zusammengefasst; angegeben sind jeweils die EC50-Werte in % (w/v).

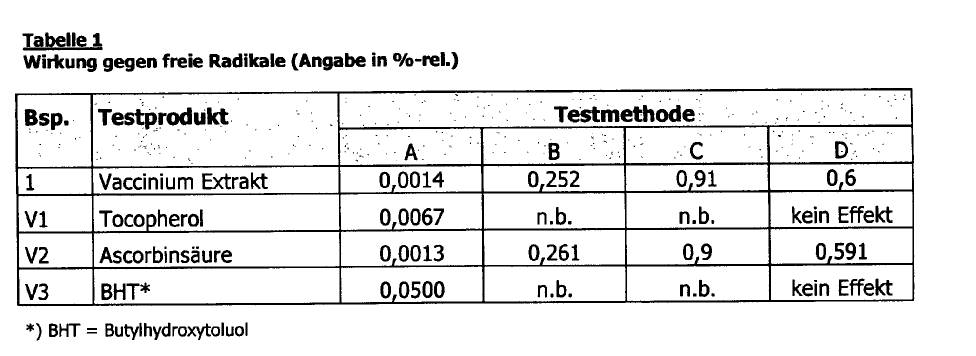

Die Ergebnisse lassen den Schluss zu, dass der Extrakt von *Vaccinium myrtyllus* eine antioxidative Wirksamkeit besitzt, die deutlich über der von Tocopherol und BHT und in der gleichen Größenordnung wie Vitamin C liegt. Die Testsubstanz besitzt in Abwesenheit von EDTA ein besonders hohes Potential zum Quenchen von Hydroxylionen, wodurch nachgewiesen ist, dass sie stabile Eisenkomplexe bilden. Schließlich besitzt sie ein starkes Potential, die Reduktion von BT durch Superoxidanionen zu verhindern.

### B. Zellschutz gegenüber UV-A-Strahlen

Gegenstand der folgenden in-vitro Untersuchungen war festzustellen, ob Vaccinium-Extrakt menschliche Fibroblasten vor oxidativem Stress, speziell der Einwirkung von UVA-Strahlen schützen kann. UVA wurde deswegen als Stressfaktor gewählt, da die Strahlen bis in die Dermis eindringen und dort vor allem eine Lipoperoxdation der Cytoplasmamembranen bewirken. Die gebildeten Lipoperoxide zerfallen in Malonaldialdehyde (MDA), die für die Retikulation vieler Biomoleküle, wie z.B. Proteine (Enzyminhibierung) oder Nucleinbasen (Mutagenese) verantwortlich sind. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVA-Strahlungsmenge geschädigt (3-15 J/cm²). Nach dem Ende der Bestrahlung wurde die Menge an gebildetem MDA in der überstehenden Lösung durch Umsetzung mit Thiobarbitursäure und der Gehalt an Proteinen im Zellhomogenisat nach der Bradford-Methode bestimmt. Die Ergebnisse sind in Tabelle 2 zusammengefasst. Die verstehen sich als %-rel gegenüber dem Standard. Angegeben ist der Mittelwert von zwei Messreihen mit Dreifachbestimmung.

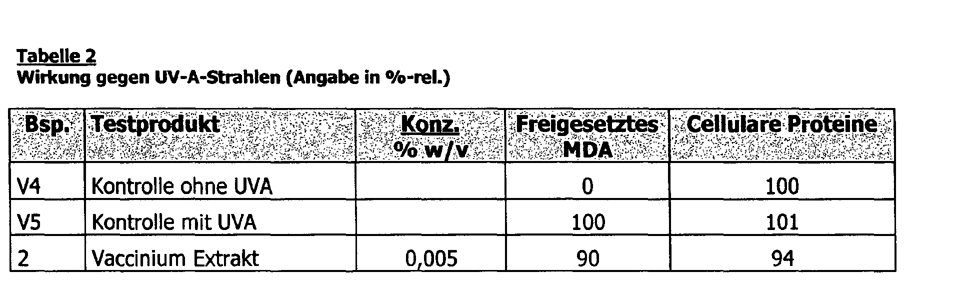

Die Ergebnisse zeigen, dass der Vaccinium-Extrakt einen nachhaltig positiven Einfluss bei der Bekämpfung von oxidativem Stress besitzt, ohne dabei die Fibroblasten zu schädigen.

### C. Zellschutz gegenüber UV-B-Strahlen

Aufgabe dieses Tests war es zu zeigen, dass Vaccinium-Extrakt anti-inflammatorische Eigenschaften gegenüber menschlichen Keratinocyten besitzen. UVB wurde als Stressfaktor ausgewählt, weil die Strahlen durch Aktivierung von Arachidonsäure freisetzenden Enzymen, wie beispielsweise Phospholipase A2 (PLA2) eine cutane Inflammation (Erytheme, Ödeme) hervorrufen. Dies führt in der Folge nicht nur zu einer Schädigung der Membranen, sondern auch zur Bildung von inflammatorisch wirkenden Stoffen, wie beispielsweise Prostaglandinen vom Typ PGE2. Der Einfluss der UVB-Strahlen auf die Keratinocyten wurde in-vitro über die Freisetzung von cytoplasmatischen Enzymen, wie z.B. LDH (Lactat Dehydrogenase) bestimmt, die parallel zur Zellschädigung und der Bildung von PGE2 verläuft. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Keratinocytenmenge wurde nach Trypsination über einen Zellzähler ermittelt, die LDH-Konzentration enzymatisch bestimmt. Die Ergebnisse sind in Tabelle 3 zusammengefasst. Angegeben ist die Aktivität in %-rel gegen einen Standard als Mittelwert von zwei Testreihen mit Doppelbestimmung.

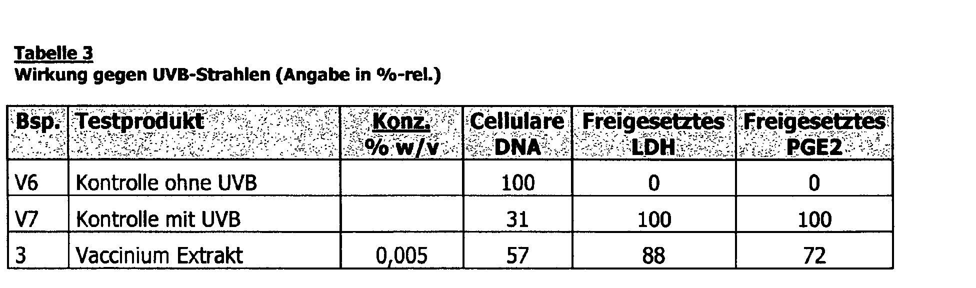

Die Ergebnisse zeigen, dass die Testsubstanzen die schädigenden Einflüsse von UVB-Strahlen signifikant reduzieren und insbesondere die Freisetzung von LDH und PGE2 vermindern.

### D. Wirksamkeit gegen Proteasen

Während einer Inflammation, werden aus den polymorphonuclearen neutrophilen Granulocyten oder Makrophagen Hautproteasen, wie beispielsweise Collagenase freigesetzt. Ein ähnlicher Vorgang spielt sich gerade in der Haut älterer Menschen bei Einfluss von UV-Strahlen ab. Die Proteasen - wegen ihres Gehaltes an zentralen Zinkionen auch als Matrix-Metallo-Proteasen (MMP) bezeichnet - katalysieren wie schon erwähnt die Fragmentierung von Bindegewebsproteinen. Zur Untersuchung der Testsubstanzen auf Collagenaseinhibierung wurde bakterielle Collagenase (clostridium histolyticum) auf Gelatine als natürlichem Nährboden verwendet, welche mit Fluorochrom (FITC, Calbiochem) markiert war. Die Inkubationszeit betrug 60 min bei 20 °C, die Hydrolyse des Substrates wurde über die Fluoreszenz bei 393 nm (Anregung bei 328 nm) verfolgt. Die Ergebnisse sind in Tabelle 4 zusammengefasst. Angegeben ist die Collagenase-Inhibierung in %.

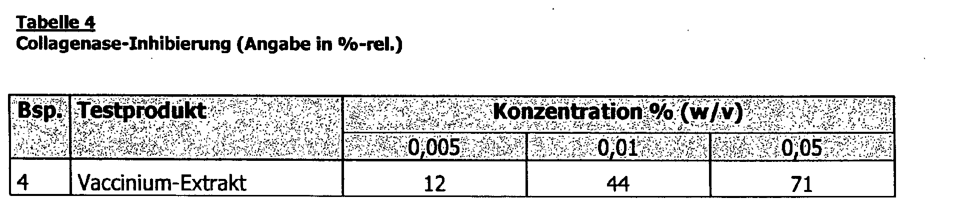

Die Ergebnisse zeigen, dass der Vaccinium-Extrakt in Abhängigkeit der Konzentration über eine signifikante Inhibierungswirkung verfügt.

### E. Inhibierung der menschlichen MMP-1 Synthese

Untersucht wurde die Fähigkeit von Vaccinium-Extrakt den toxischen Einfluss von UVA-Strahlen zu mindern. Als in-vitro System diente eine Kultur dermaler Fibroblasten, bestimmt wurde die Freisetzung von MMP-1 aus diesen Fibroblasten unter dem Einfluss der UV-Strahlung. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Bestimmung der MMP erfolgte mit einem Kit, das unter der Bezeichnung RPN2610 von der Firma Amersham im Handel erhältlich ist. Die Ergebnisse sind in Tabelle 5 zusammengefasst. Angegeben ist die Menge MMP in ng/ml aus einer Testreihe mit Dreifachbestimmung.

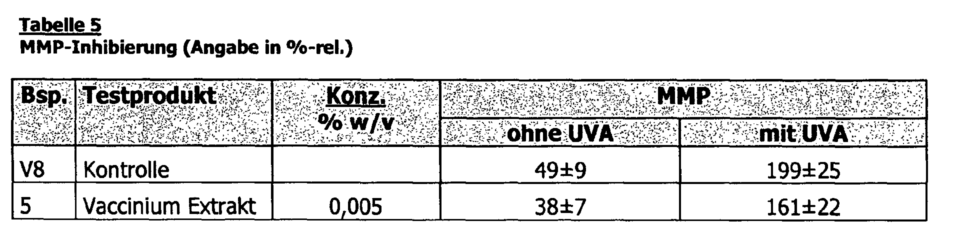

Die Ergebnisse zeigen, dass Vaccinium-Extrakt die Freisetzung von MMP bei UVA-Bestrahlung nachhaltig vermindert.

### F. Anti-inflammatorische Wirkung

Im Verlauf einer cutanen Inflammation werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur proinflammatorische Cytokine, Leukotriene und Proteasen, sondern auch ROS, wie z.B. Superoxide und Hypochloritanionen frei, welche die Aufgabe haben, eingedrungene pathogene Keime oder Pilze zu vernichten. Diese Aktivität der PMN während der Inflammation ist als sogenannter Atmungsausbruch ("respiratory burst") bekannt und kann zu zusätzlichen Schäden im Gewebe führen. Zur Untersuchung, inwiefern OPC und OPC-reiche Extrakte den Atmungsausbruch verhindern oder mindern können, wurde eine Zelllinie von menschlichen leukemischen Granulocyten dieser PMN zusammen mit den Testsubstanzen bei 37 °C von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 6 zusammengefasst. Angegeben sind die Zellzahlen sowie die Menge an freigesetzten ROS in %-rel zum Standard als Mittelwert einer Messreihe mit Dreifachbestimmung.

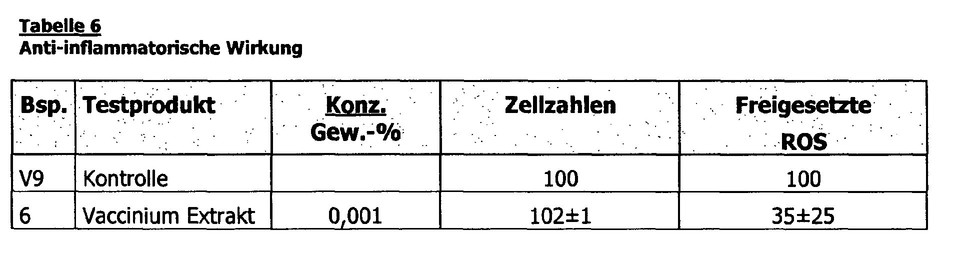

Die Ergebnisse zeigen, dass Vaccinium-Extrakt einen starken inhibierenden Einfluss auf den Atmungsausbruch menschlicher Granulocyten besitzt, ohne die Granulocyten zu schädigen.

### G. Regenerative und wachstumsstimulierende Wirkung

Nach einer Inkubation von 72 h in einer Nährlösung bilden Fibroblasten gesättigte Monolayer aus, die Fibroblasten stellen ihre Aktivität ein und das Wachstum kommt zum Stillstand. Der Zellbrennstoff Adenosintriphosphat (ATP), der im wesentlichen in den Mitochondrien entsteht, wird zur Aktivierung von bestimmten Enzymen benötigt, die beispielsweise das Zellskelett kontrollieren, die Ionenkanäle, die Aufnahme von Nährstoffe und eine ganze Reihe anderer wichtiger biologischer Abläufe. Die Bestimmung des Proteingehaltes in den Zellen erfolgte nach der Methode von Bradford [ vgl. **Anal. Biochem. 72, 248-254 (1977) ].** Glutathion (GSH) ist ein spezielles Protein, welches von den Zellen zum Schutz gegen oxidativen Stress und Umweltgifte, insbesondere gegen Schwermetalle produziert wird. Die an der reduzierten Form des GSH beteiligten drei Aminosäuren sind mit speziellen cytoplasmatischen Enzymen verknüpft, die zur Aktivierung ATP benötigen. Ein Anstieg der GSH-Konzentration führt zu einem Anstieg der Glutathion-S-transferase-Aktivität, eines Entgiftungsenzyms. Der GHS-Gehalt wurde nach der Methode von Hissin [vgl. **Anal. Biochem. 74, 214-226 (1977)]** bestimmt. Die *wachstumsstimulierende Wirkung* von Vaccinium-Extrakt wurde an menschlichen Fibroblasten untersucht. Dazu wurden in einer ersten Testreihe die Fibroblasten in einem Nährmedium 1 Tag bei 37 °C und 5 Vol.-% CO₂ inkubiert, das Nährmedium gegen ein Medium, welches die Testsubstanz enthielt, ausgewechselt und wiederum 3 Tage bei 37 °C inkubiert. Anschließend wurde der Proteingehalt in den Zellen sowie die ATP-Konzentration bestimmt. Zur Bestimmung der *überlebensstimulierenden Wir* *kung* wurden in einer zweiten Testreihe die Fibroblasten zunächst 3 Tage bei 37 °C in einer Nährlösung und dann 3 Tage bei gleicher Temperatur in einer Testlösung inkubiert. Anschließend wurde der Proteingehalt in den Zellen sowie die GSH-Konzentration bestimmt.
Die Ergebnisse sind in Tabelle 7 zusammengefasst. Angegeben sind die Ergebnisse von jeweils 3 Messreihen mit Dreifachbestimmung in %-rel gegenüber einer Blindprobe.

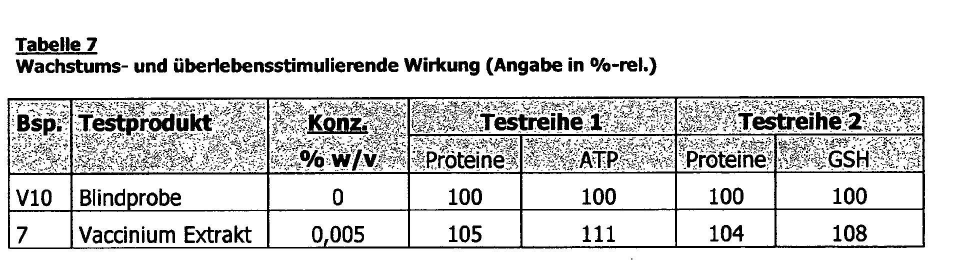

Die Beispiele zeigen, dass die Testsubstanzen den Metabolismus im Hinblick auf das Wachstum und den Schutz der Fibroblasten stimulieren.

### H. Wirksamkeit gegen Hautalterung

Das Enzym Glucose-6-phosphat Dehydrogenase (G6PDH) katalysiert den ersten Schritt des sogenannten "Pentose-Wegs", bei dem ein wesentlicher DNA-Baustein, nämlich die Desoxyribose gebildet wird. Dabei wird Glucose-6-phosphat (G6P) mit Hilfe von G6PDH in 6-Phosphatogluconat (6PG) überführt. Gleichzeitig wird das dabei erforderliche Coenzym NADP wird zu NADPH2 reduziert, welches seinerseits eine Vielzahl von anderen biologischen Reaktion wie z.B. das Recycling von Glutathion oder die Lipidsynthese katalysieren kann. Reduziertes Glutathion schützt viele Enzyme, die über SH-Gruppen verfügen und Zellen vor oxidativem Stress, wie beispielsweise UV-Einwirkung. Damit ist der G6PDH-Gehalt ein wichtiger Parameter für den Zellschutz und die die Hauterneuerung. Die G6PDH-Aktivität wurde in-vitro an menschlichen Fibroblasten nach der enzymatischen Methode von Okada bestimmt, der DNA-Gehalt nach dem Verfahren von Desaulniers. Die Ergebnisse sind in Tabelle 8 zusammengefasst. Angegeben sind die Ergebnisse von jeweils 3 Messreihen mit Dreifachbestimmung in %-rel gegenüber einer Blindprobe.

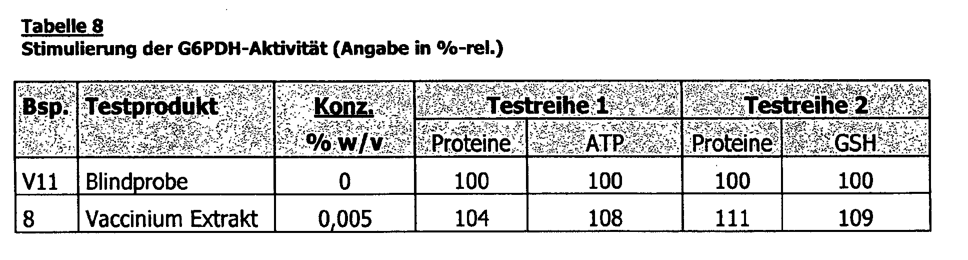

### I. Immunstimulation

Unter Immunstimulation sind biochemische Abläufe zu verstehen, bei denen Botenstoffe, wie beispielsweise Betaglucane die körpereigenen Abwehrkräfte stimulieren, um beispielsweise toxische Stoffe zu binden und auszuscheiden und die Erneuerung von Hautzellen zu beschleunigen. Es ist bekannt, dass Organismen diese Fähigkeit mit zunehmendem Alter verloren geht. Die Immunstimulation kann in-vitro an Hand von menschlichen Leukocyten beobachtet werden, welche zuvor mit einem Hefeextrakt (Zymosan) aktiviert worden sind [vgl. Capsoni et al. Int.J.Immunopharm. 10(2), 121-133 (1998)]. Eine Kultur polymorphonuclearer neutrophiler Granulocyten (PMN) wurde zusammen mit Vaccinium-Extrakt über einen Zeitraum von 24 h bei 37 °C und 5 Vol.-% CO₂ inkubiert. Durch Zugabe von Zymosan wurde der Atmungsausbruch ausgelöst. Nach 30 min wurde die PMN-Zahl durch einen automatischen Zellzähler und die Menge an freigesetzten reaktiven Sauerstoffspezies (ROS) in der überstehenden Flüssigkeit mit Hilfe von Luminol spektroskopisch bestimmt. Die Ergebnisse sind in Tabelle 9 zusammengefasst und verstehen sich als %-rel gegenüber dem Standard. Angegeben ist der Mittelwert von zwei Messreihen mit Dreifachbestimmung.

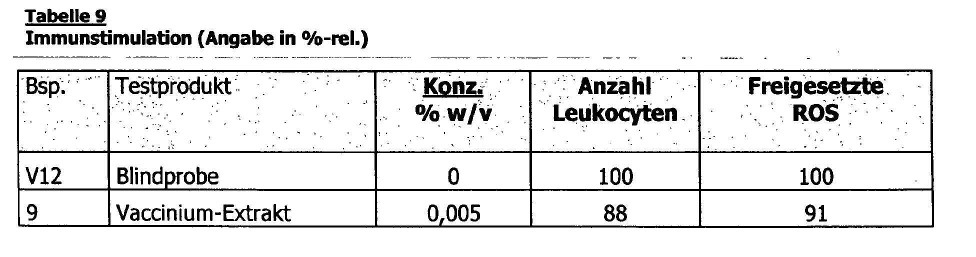

Die Ergebnisse zeigen, dass die Testsubstanzen das Immunsystem stimulieren und die körpereigenen Abwehrkräfte insbesondere der Hautzellen nachhaltig stärken.

### J. Aktivierung der Lipolyse

Unter Lipolyse versteht man die Entfernung von Triglyceriden aus den Adipocyten. Wesentliche Bedeutung kommt dabei der Triglycerid Lipase zu, die die Glyceride in freie Fettsäuren und Glycerin spaltet, die dann über den Blutkreislauf abtransportiert werden. Die Fettsäuren können dann beispielsweise in den Muskelzellen verbrannt werden und dienen somit als Energiespeicher. Zur Untersuchung der lipolytischen Aktivität wurden Adipocyten aus menschlichem subcutanem Gewebe isoliert [vgl. Rodbell, J.Biol.Chem. 239(2), 375-380 (1964)]. Die entsprechenden Kulturen wurden mit den Testsubstanzen geimpft und über einen Zeitraum von 90 min bei 37 °C inkubiert. Die Menge an freigesetztem Glycerin in der überstehenden Flüssigkeit wurde spektroskopisch bestimmt [vgl. Carpéné et al., J.Pharmacol. (Paris), 12(2), 219-224 (1981)]. Die Ergebnisse sind in Tabelle 10 zusammengefasst und verstehen sich als %-rel gegenüber dem Standard. Angegeben ist der Mittelwert von zwei Messreihen mit Dreifachbestimmung.

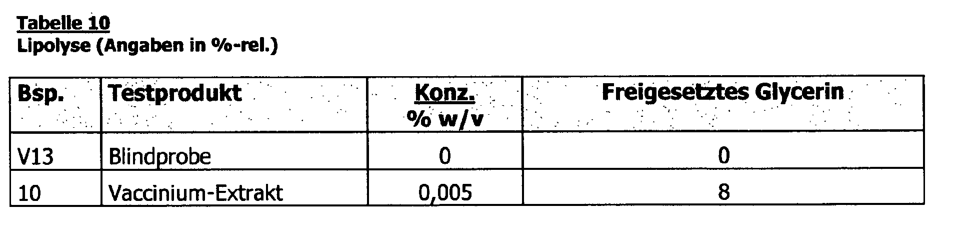

Die Ergebnisse zeigen, dass die Testsubstanzen die Lipolyse anregen und damit zu einer schnelleren Verfügbarkeit von Zellbrennstoff beitragen.

In Tabelle 11 sind verschiedene Rezepturen für Hautbehandlungsmittel mit Vaccinium-Extrakten aufgeführt.

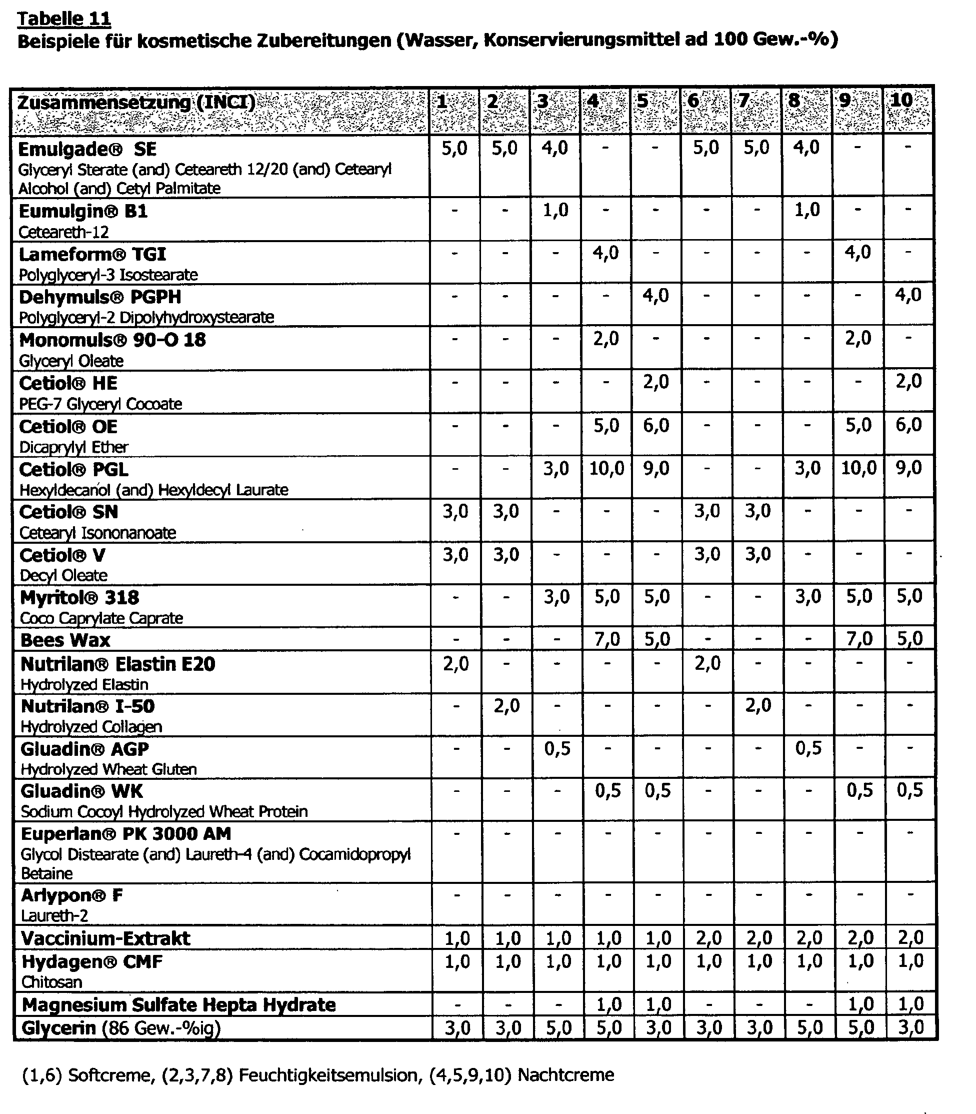

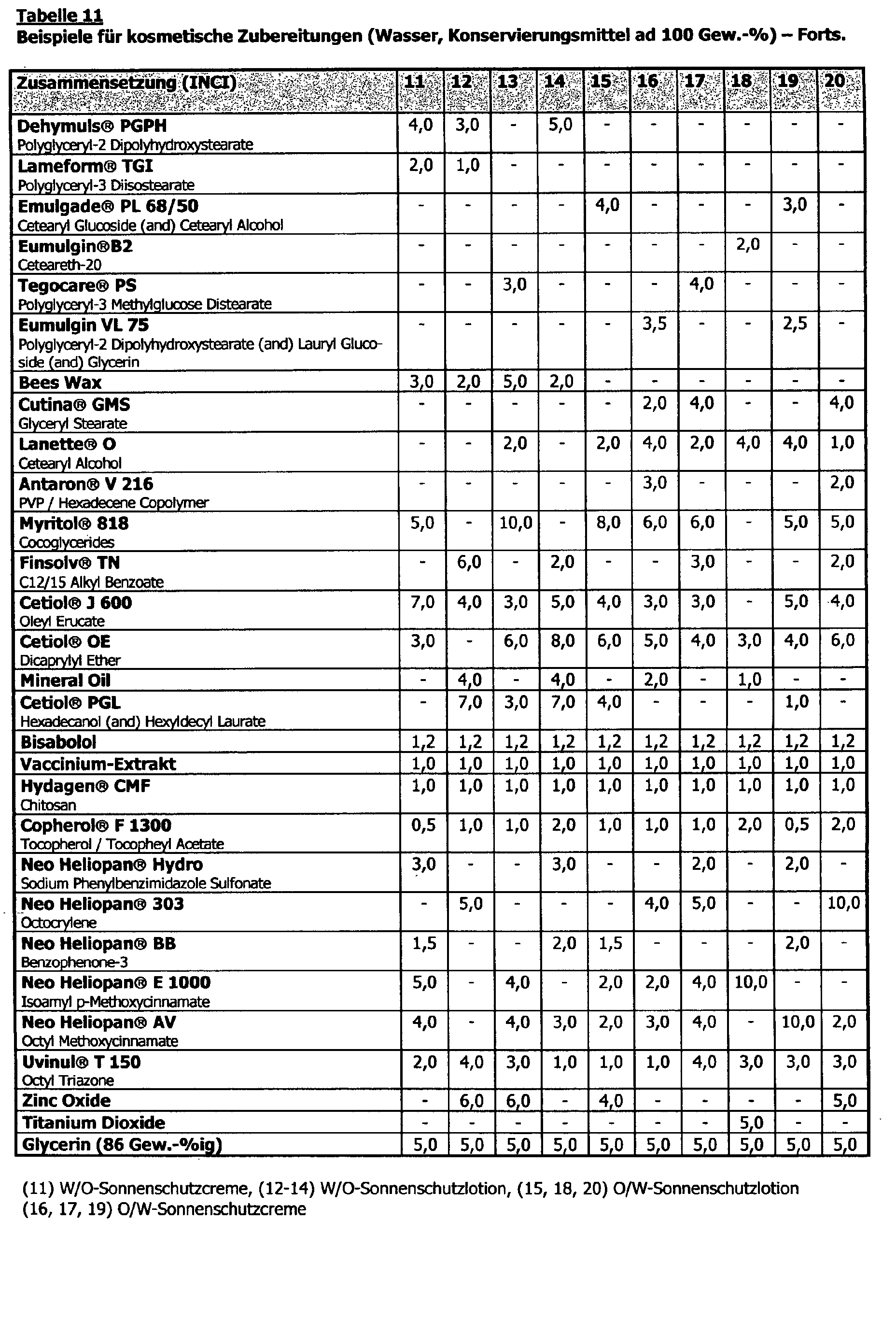

## Patentansprüche

1. Anti-Ageing-Mittel, **dadurch gekennzeichnet, dass** sie einen wirksamen Gehalt an Pflanzenextrakten der Gattung Vaccinium enthalten.

2. Verwendung von Pflanzenextrakten der Gattung Vaccinium als Mittel zum Schutz von Haut und Haaren gegen Alterung und Umwelteinflüsse.

3. Verwendung nach Anspruch 2, **dadurch gekennzeichnet, dass** man Extrakte von *Vaccinium myrtyllus* einsetzt.

4. Verwendung nach mindestens einem der Ansprüche 2 bis 3, **dadurch gekennzeichnet, dass** sie Haut und Haare gegen den Einfluss freier Radikale schützen.

5. Verwendung nach mindestens einem der Ansprüche 2 bis 4, **dadurch gekennzeichnet, dass** sie Haut und Haare vor dem Einfluss von UV-A und UV-B-Strahlung schützen.

6. Verwendung nach mindestens einem der Ansprüche 2 bis 5, **dadurch gekennzeichnet, dass** sie die Haut vor dem Einfluss von Proteasen und insbesondere Collagenasen schützen.

7. Verwendung von Pflanzenextrakten der Gattung Vaccinium als MMP-Inhibitoren.

8. Verwendung von Pflanzenextrakten der Gattung Vaccinium als anti-inflammatorische Wirkstoffe.

9. Verwendung von Pflanzenextrakten der Gattung Vaccinium als regenerative und wachstumsstimulierende Faktoren in Hautbehandlungsmitteln.

10. Verwendung von Pflanzenextrakten der Gattung Vaccinium zur Stimulierung der Synthese von Glucose-6-phosphat Dehydrogenase (G6PDH).

11. Verwendung von Pflanzenextrakten der Gattung Vaccinium zur Stimulation des Immunsystems der Haut.

12. Verwendung von Pflanzenextrakten der Gattung Vaccinium zur Aktivierung der Lipolyse von Fetten in der Haut.
